# EUROPEAN PATENT APPLICATION

(11) **EP 4 015 637 A1**
(43) Date of publication of application: **22.06.2022**
(21) Application number: 20215905.9
(22) Date of filing: 21.12.2020
(51) Int. Cl.: C12N 15/86

(54) **PRODUCER CELL LINE HAVING LOW LEVELS OF VA RNA**

(71) Applicant: CEVEC Pharmaceuticals GmbH, 51105 Köln (DE)
(72) Inventor: HEIN, Kerstin, 50968 Köln (DE); DO CARMO GIL GONCALVES, Ines, 50825 Köln (DE); WISSING, Silke, 35037 Marburg (DE); HUDJETZ, Ben, 50226 Frechen (DE)
(74) Representative: Müller-Boré & Partner Patentanwälte PartG mbB

(57) **Abstract**

The present invention relates to host cells comprising a nucleic acid encoding an Adeno-associated virus (AAV) capsid protein, wherein the protein kinase R (PKR) activating sequence surrounding the start codon of AAV capsid protein VP1 is inactivated, while maintaining functional expression of the AAV capsid proteins VP1, VP2, and VP3. The present invention further relates to methods for the production of Adeno-associated virus (AAV), comprising the step of expressing said AAV in the host cells of the present invention.

## Description

The present invention relates to host cells comprising a nucleic acid encoding an Adeno-associated virus (AAV) capsid protein, wherein the protein kinase R (PKR) activating sequence surrounding the start codon of AAV capsid protein VP1 is inactivated, while maintaining functional expression of the AAV capsid proteins VP1, VP2, and VP3. The present invention further relates to methods for the production of Adeno-associated virus (AAV), comprising the step of expressing said AAV in the host cells of the present invention.

Adeno-associated viruses (AAV) are small viruses that infect humans and some other primate species. They belong to the genus *Dependoparvovirus,* which in turn belongs to the family *Parvoviridae.* They are small (20 nm) replication-defective, non-enveloped viruses.

AAV are not currently known to cause any disease and elicit only a very mild immune response. Several additional features make AAV an attractive candidate for creating viral vectors for gene therapy, and for the creation of isogenic human disease models. Gene therapy vectors using AAV can infect both dividing and quiescent cells and persist in an extrachromosomal state without integrating into the genome of the host cell, although in the native virus, integration of virally carried genes into the host genome does occur.

Recently, there has been a rapid increase in the number of approved products as well as gene therapy clinical trials based on AAV-derived vectors. Advantages of AAV vectors in gene therapy are an excellent safety profile, the above-mentioned non-pathogenicity, a stable expression of transgenes, and the possibility of transducing dividing and non-dividing cells.

The production of recombinant AAV currently requires a number of components. Replicase (Rep) and capsid (Cap) proteins, usually encoded by the AAV-genome, are needed for the production of recombinant virus and are supplied *in trans.* Helper genes can be derived from different helper viruses, the most common being the helper virus genes E1A, E1B, E2A, E4orf6, and VA RNA (viral associated RNA) taken from Adenovirus. In addition, a transfer vector containing the gene of interest (GOI) flanked by inverted terminal repeat sequences (ITRs) is needed.

Current AAV-based production systems rely mostly on the following techniques which, however, have several drawbacks. Transient transfection usually requires a two- or three-plasmid system (transfer vector containing gene of interest; pHelper with adenoviral helper functions; pAAV-Rep2CapX (CapX = capsid function of different AAV serotypes) supplying the capsid and replicase functions). However, such techniques lack sufficient scalability, robustness, and reproducibility, and entail high costs for plasmid DNA. So-called producer cell lines with an already stably integrated gene of interest are mostly based on HEK or HeLa cells. However, such cells need additional infection with helper virus, e.g. Adenovirus. This addition of helper virus during AAV production requires first production of the corresponding helper virus, then extensive purification of the produced AAV vector, in order to remove the helper virus from the final product, and costly proof of absence of helper viruses. The same applies for Herpes simplex virus (HSV)- and Baculovirus-based systems, the latter of which additionally lack sufficient scalability and robustness.

Various difficulties hamper the generation of fully stable AAV-based producer cell lines, in which all components necessary for the production of AAV are stably integrated in the cell. Integration of all helper functions into cells is challenging. In CAP or HEK293 cells, E1A and E1B are already constitutively expressed. The helper functions E2A and E4orf6 are toxic to the cells. However, these helper functions can be expressed from inducible promoters. This is more difficult for non-coding VA RNA, which has its PolIII promoter located within its own coding sequence.

VA RNA is a non-coding RNA found in adenovirus. It plays a role in regulating translation. There are two copies of this RNA called VAI or VA RNAI and VAII or VA RNAII. These two VA RNA genes are distinct genes in the adenovirus genome. VA RNAI is the major species with VA RNAII expressed at a lower level. VAI stimulates the translation of both early and late adenoviral genes including E3 and hexon. Transient transfection assays have shown that VAI increases the stability of ribosome-bound transcripts. Further, VAI is processed in the cell to create 22 nucleotides long RNAs that can act as siRNA or miRNA. Moreover, VAI functions as a decoy RNA for the double stranded RNA-activated protein kinase R which would otherwise phosphorylate eukaryotic initiation factor 2 (eIF2).

In the context of AAV, VA RNA is important for high-titer AAV production. The best described function of the VA RNA within AAV production is the suppression of PKR phosphorylation/activation and thereby, elF2alpha phosphorylation, which leads to a reduction of viral protein expression. elF2alpha is a subunit of the trimeric eukaryotic initiation factor 2 (eIF2) that regulates global translation by binding to Met-tRNA and the 40S ribosome to form the pre-initiation complex. Upon phosphorylation at serine 51, exchange of the eIF2-bound GDP to GTP by the guanine nucleotide exchange factor (GEF) eIF2B is blocked resulting in an inhibition of protein synthesis. It has been shown that PKR is activated by the short region of the capsid gene RNA leader sequence surrounding the AUG of VP1. This sequence is part of the intron between the p40 promoter, which is embedded into the upstream rep gene, and the capsid gene. Inhibition of PKR activity is a common mechanism occurring upon infection of a number of different viruses such as e.g. hepatitis C virus.

In this context, Nayak and Pintel (Nayak, R. and Pintel, D. J.; Journal of Virology, 81(21); 2007; pp. 11908-11916) describe how VA RNA during the AAV infection cycle is important for capsid expression, *i.e.,* how capsid pre-mRNA is activating PKR, which in turn phosphorylates eIF2α, which in turn leads to low expression of capsid proteins. Further, said document tries to identify PKR activating sequences by deletion experiments and narrows the same down to about 200 nucleotides around the AUG sequence of VP1.

In summary, current AAV-based production systems are limited with respect to their reproducibility, scalability and robustness. Further, the requirement for helper viruses necessitates extensive purification and the costly proof of absence of helper virus.

Accordingly, the technical problem underlying the present invention is the provision of a scalable system for the stable production of AAV vectors that does not require transient transfection or helper viruses, allowing for the industrial and scalable production of AAV gene therapy vectors.

The solution to the above technical problem is achieved by the embodiments characterized in the claims.

In particular, in a first aspect, the present invention relates to a host cell comprising a nucleic acid encoding Adeno-associated virus (AAV) capsid protein, wherein the protein kinase R (PKR) activating sequence surrounding the start codon of AAV capsid protein VP1 is inactivated, while maintaining functional expression of the AAV capsid proteins VP1, VP2, and VP3.

AAV in the present invention is not limited to particular AAV serotypes. Thus, AAV can be selected from Adeno-associated virus serotype 1 (AAV1), AAV2, AAV3, AAV4, AAV5, AAV6, AAV7, AAV8, AAV9, AAV10, AAV11, AAVDJ, AAVDJ8, and AAVrh10. Further, AAV can be a synthetic serotype, e.g. a hybrid of two or more different of the above serotypes, or one of the above serotypes with significant mutations altering the tropism of the AAV serotype. However, in particular embodiments, AAV is AAV2, AAV5, AAV6, AAV8, or AAV9, preferably AAV8.

AAV capsid protein encompasses three capsid proteins, VP1, VP2, and VP3, which are natively expressed from one promoter, designated p40, and are encoded by the cap gene. The molecular weights of these proteins are 87, 72 and 62 kDa, respectively. The AAV capsid is composed of a mixture of VP1, VP2, and VP3 totaling 60 monomers arranged in icosahedral symmetry in a ratio of 1:1:10, with an estimated size of 3.9 MDa.

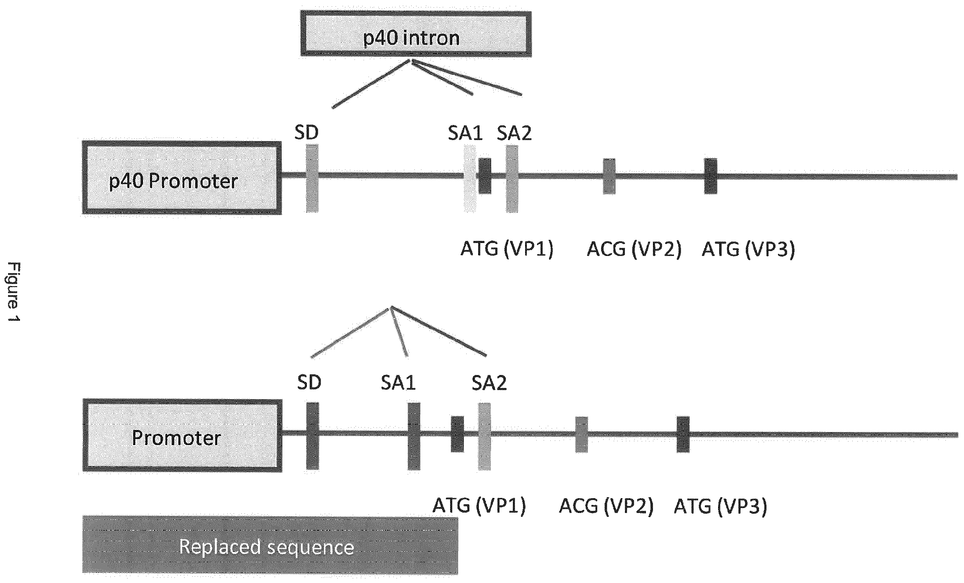

All three VPs are translated from one mRNA. After pre-mRNA is synthesized, it can be spliced in two different manners due to alternative splicing at two acceptor sites (Fig. 1). Usually, especially in the presence of adenovirus, the second splice acceptor site is preferred and represents the so-called "major splice". In this form, the first AUG codon, from which the synthesis of VP1 protein starts, is cut out, resulting in a reduced overall level of VP1 protein synthesis. The first AUG codon that remains in the major splice is the initiation codon for VP3 protein. However, upstream of that codon in the same open reading frame there is an ACG sequence (encoding threonine) which is surrounded by an optimal Kozak context. This contributes to a low level of synthesis of VP2 protein, which consists of the VP3 protein with additional N terminal residues.

Since the larger intron is preferred to be spliced out, and since in the major splice the ACG codon is a much weaker translation initiation signal, the ratio at which the AAV structural proteins are synthesized *in vivo* is about 1:1:10, which is the same as in the mature virus particle.

In the capsid protein pre-mRNA, a short sequence surrounding the AUG of VP1 present in the intron transcribed by the p40 promoter, activates PKR. Active PKR, in turn, phosphorylates eIF2α, wherein phosphorylated eIF2α, in turn, suppresses capsid protein expression, the entire system constituting a regulatory negative feedback loop. VA RNA suppresses PKR, explaining the necessity for high levels of VA RNA when attempting to express AAV in high amounts.

Nayak and Pintel have shown that the requirement of VA RNA can be abolished by deleting the sequence surrounding the initial AUG. However, this also leads to a loss of expression of the VP1 protein. Nayak and Pintel do not suggest a solution towards expressing all three capsid proteins at the correct ratio. Neither do they suggest to make use of this observation to generate a stable cell line for AAV production without VA RNA.

In order to abolish the necessity of high VA RNA levels without losing expression of VP1, the present invention advantageously found that the PKR-activating sequence surrounding the AUG of VP1 can be inactivated while maintaining functional expression of VP1, VP2, and VP3 by replacing the sequence encoding the intron of the capsid pre-mRNA partially, in particular by replacing the splice donor and splice acceptor site 1 of said intron by an intron of different origin.

In preferred embodiments, the above PKR-activating sequence is inactivated by replacing the native AAV capsid protein promoter p40 and the part of the p40 intron containing the splice donor and splice acceptor site 1 outside of the coding capsid sequence by an intron of different origin. Preferably, the native AAV capsid protein promoter p40 is replaced by a constitutive promoter or an inducible promoter, and the native AAV capsid protein promoter p40 intron is partially replaced, *i.e.,* is replaced outside of the capsid coding sequence, by an SV40 intron, replacing the splice donor and splice acceptor site 1 (Fig. 1). However, besides the SV40 intron, any intron supplying a fitting splice donor and splice acceptor site (minimal consensus sequences GT/AG) can serve as replacement intron. Respective introns are known in the art, e.g. the synthetic CAG promoter intron, or the (human) beta-globin intron.

In preferred embodiments, the respectively modified AAV capsid sequence is stably integrated into the host cell genome.

In preferred embodiments, the host cells of the present invention further comprise a nucleic acid encoding AAV replicase (Rep) proteins under the control of a constitutive promoter or an inducible promoter.

In further preferred embodiments, the host cells of the present invention further comprise a nucleic acid encoding adenoviral E1A and E1B proteins.

In this context, said E1A and E1B proteins are preferably expressed constitutively. Further, host cells suitable for the present invention are not particularly limited and are known in the art. However, in preferred embodiments, said host cells display constitutive E1A and E1B expression. In preferred embodiments, said host cells are CAP cells, HEK293 cells or Per.C6 cells, *i.e.,* are derived from said cell lines, which are all characterized by constitutive E1A and E1B expression.

In preferred embodiments, the host cells of the present invention further comprise a nucleic acid encoding adenoviral E2A and E4orf6 proteins under the control of a constitutive promoter or an inducible promoter.

Moreover, the host cells of the present invention preferably further comprise a nucleic acid encoding a transfer vector containing one or more genes of interest (GOI), which are preferably flanked by AAV inverted terminal repeat sequences (ITRs).

GOIs to be used in the context of the present invention are not particularly limited and include any genes the transfer of which into the recipient of the AAV vector is of interest, e.g. for treatment of eye diseases, blindness diseases, muscular diseases, Duchenne muscular dystrophy, GM2 gangliosidosis and spinocerebellar ataxia type, ALS, Huntington disease, X-linked severe combined immunodeficiency (X-SCID), adenosine deaminase deficiency (ADA-SCID), central nervous system diseases, Parkinson's disease, Alzheimer disease, liver diseases, liver enzyme ornithine transcarbamylase (OTC) deficiency, Leber's congenital amaurosis, hemophilia, β-thalassemia, cancer diseases, head and neck cancer, metastatic melanoma, heart diseases, lung diseases, or cystic fibrosis, Wiskott-Aldrich syndrome (WAS), metachromatic leukodystrophy (MLD), and severe lipoprotein lipase deficiency disorder (LPLD) infection diseases, severe combined immunodeficiency syndrome, HIV infection, rare diseases including Niemann Pick Disease Type C and ornithine transcarbamylase (OTC) deficiency.

Inducible promoters to be used in the context of the present invention are not particularly limited and are known in the art. However, in preferred embodiments, the inducible promoter controlling expression of the AAV capsid protein and/or the inducible promoter controlling expression of AAV replicase proteins and/or the inducible promoter controlling expression of adenoviral E2A and E4orf6 proteins, is a tet-inducible promoter such as the third generation TRE3G-promoter. Other inducible promoters that can be used in the context of the present invention include the cumate-inducible promoter, tamoxifen-inducible promoter, rapamycin-inducible promoter, FKCsA-inducible promoter, ABA-inducible promoter, riboswitch-controlled promoters, and heat shock promoter-driven, light-switchable systems. In this context, more than one or all of the above proteins can be expressed under the control of independent promoters of the same kind.

In this context, in alternative embodiments, non-inducible promoters for constitutive expression can also be used, as known in the art. Respective promoters include the CMV, EF1alpha, SV40, RSV, UbC, CAG, BOS and PGK promoters.

In specific embodiments, in the host cells of the present invention,
the PKR-activating sequence is inactivated by replacing the native AAV capsid protein promoter p40 and the part of the p40 intron containing the splice donor and splice acceptor site 1 outside of the coding capsid sequence by an intron of different origin,
wherein the native AAV capsid protein promoter p40 is replaced by a constitutive or inducible promoter, preferably an inducible promoter, and the native AAV capsid protein promoter p40 intron is partially replaced, *i.e.,* is replaced outside of the capsid coding sequence, by an intron, preferably a SV40 intron, replacing the splice donor and splice acceptor site 1, and
wherein the respective nucleotide sequence from the inducible promoter to the start codon of the coding sequence of the AAV capsid protein has the structure
Promoter - N1 - Intron - N2 - ATG
wherein
Promoter is the constitutive or inducible promoter, preferably the inducible promoter,
N1 is a sequence of 1 to 4000 nucleotides,
Intron is an intron, selected from the group consisting of introns supplying a fitting splice donor and splice acceptor site with the minimal consensus sequences GT/AG,
N2 is a sequence of 1 to 4000 nucleotides, and
ATG is the start codon of the AAV capsid protein coding sequence.

Preferably, in the above structure, Promoter is a constitutive promoter, selected from the group consisting of the CMV, EF1alpha, SV40, RSV, UbC, CAG, BOS and PGK promoters, or an inducible promoter, selected from the group consisting of a tet-inducible promoter such as the third generation TRE3G-promoter, a cumate-inducible promoter, a tamoxifen-inducible promoter, a rapamycin-inducible promoter, an FKCsA-inducible promoter, an ABA-inducible promoter, riboswitch-controlled promoters, and heat shock promoter-driven, light-switchable systems, wherein inducible promoters, in particular tet-inducible promoters, are particularly preferred. Further, in the above structure, Intron is preferably an intron, selected from the group consisting of the SV40 intron, the synthetic CAG promoter intron, and the (human) beta-globin intron. Furthermore, N1 and N2, independently from each other, are preferably a sequence of 1 to 3000, more preferably 1 to 2000, more preferably 1 to 1000, more preferably 1 to 500, more preferably 1 to 300 nucleotides. In further embodiments, N1 and N2, independently from each other, are preferably a sequence of 50 to 250, or 100 to 200 nucleotides.

In more specific embodiments, in the host cells of the present invention,
the PKR-activating sequence is inactivated by replacing the native AAV capsid protein promoter p40 and the part of the p40 intron containing the splice donor and splice acceptor site 1 outside of the coding capsid sequence by an intron of different origin,
wherein the native AAV capsid protein promoter p40 is replaced by an inducible promoter, and the native AAV capsid protein promoter p40 intron is partially replaced, *i.e*., is replaced outside of the capsid coding sequence, by an SV40 intron, replacing the splice donor and splice acceptor site 1, and
wherein the respective nucleotide sequence from the inducible promoter to the start codon of the coding sequence of the AAV capsid protein is
(i) the nucleotide sequence according to SEQ ID NO: 4 or SEQ ID NO: 5, or
(ii) a nucleotide sequence having at least 70% sequence identity to SEQ ID NO: 4 or SEQ ID NO: 5, provided the above prerequisites apply to said nucleotide sequence,
wherein the last three nucleotides ATG of SEQ ID NO: 4 or SEQ ID NO: 5 are the start codon of the AAV capsid protein coding sequence.

As indicated above, nucleic acids having sequence identity to the specific nucleotide sequences indicated above, have at least 70% sequence identity to the specific sequences. Preferably, said nucleotide sequences have at least 72%, 74%, 76%, 78%, 80%, 82%, 84%, 86%, 88%, 90%, 91%, 92%, 93%, 94%, 95%, 95.5%, 96%, 96.5%, 97%, 97.5%, 98%, 98.2%, 98.4%, 98.6%, 98.8%, 99%, 99.1%, 99.2%, 99.3%, 99.4%, 99.5%, 99.6%, 99.7%, 99.8%, or 99.9% sequence identity to a nucleic acid as defined above. In particular embodiments, such nucleic acids have 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 nucleotide deletions, insertions and/or replacements (exchanges).

Respective nucleic acids having a defined sequence identity to a nucleic acid as defined above or having specific nucleotide deletions, insertions and/or replacements with respect to a nucleic acid as defined above exclude any nucleic acids having any kind of frameshift mutation.

Further, the term "functional" as used above in the context of proteins that are encoded by nucleotide sequences having a certain sequence identity to the specific nucleotide sequences indicated above, indicates that the respective proteins have the same or at least sufficient activity or function as compared to proteins encoded by the specific nucleotide sequences.

The nucleic acid comprised in the host cell of the present invention can further comprise one or more elements, selected from the group consisting of inducible promoters, poly(A) regions, selection markers, IRES sequences and enhancing elements. Suitable inducible promoters are not particularly limited and are known in the art, e.g. Tet-inducible promoters such as the third generation TRE3G-promoter. Suitable poly(A) regions are not particularly limited and are known in the art, e.g. the SV40 poly(A) region. Suitable selection markers are not particularly limited and are known in the art, e.g. antibiotic resistance cassettes such as blasticidin or ampicillin resistance cassettes.

Preferably, in the host cells of the present invention, the nucleic acid encoding an AAV capsid protein, and/or the nucleic acid encoding AAV replicase proteins, and/or the nucleic acid encoding adenoviral E1A and E1B proteins, and/or the nucleic acid encoding adenoviral E2A and E4orf6 proteins, and/or the nucleic acid encoding the transfer vector containing a GOI, is/are stably integrated into the host cell genome.

Preferably, the host cells of the present invention do not express VA RNA, or express only low levels of VA RNA, e.g. at least 10-fold, preferably at least 100-fold, more preferably at least 1000-fold reduced VA RNA expression levels as compared to VA RNA expression achieved by transient transfection.

Methods for generating the host cells of the present invention, *i.e.,* methods for the introduction of the nucleic acids of the present invention into suitable host cells, are not particularly limited and are known in the art.

In a second aspect, the present invention relates to a method for the production of Adeno-associated virus (AAV), comprising the step of expressing said AAV in a host cell according to the present invention.

In a third aspect, the present invention relates to the use of a host cell according to the present invention in the production of Adeno-associated virus (AAV).

Respective means for producing AAV are not particularly limited and are known in the art.

Specifically, in preferred embodiments, the methods and uses of the present invention encompass the step of causing the host cells of the present invention to express (i) AAV capsid protein, wherein, according to the present invention, the protein kinase R (PKR) activating sequence surrounding the start codon of AAV capsid protein VP1 is inactivated, while maintaining functional expression of the AAV capsid proteins VP1, VP2, and VP3. Preferably, the PKR-activating sequence surrounding the AUG of VP1 is inactivated by replacing the intron of the capsid pre-mRNA partially, in particular by replacing the splice donor and splice acceptor site 1 of said intron, by an intron of different origin. In preferred embodiments, the above PKR-activating sequence is inactivated by replacing the native AAV capsid protein promoter p40 and the part of the p40 intron containing the splice donor and splice acceptor site 1 outside of the coding capsid sequence by an intron of different origin. Preferably, the native AAV capsid protein promoter p40 is replaced by an inducible promoter, and/or the native AAV capsid protein promoter p40 intron is partially replaced, *i.e*., is replaced outside of the capsid coding sequence, by an SV40 intron, replacing the splice donor and splice acceptor site 1 (Fig. 1). However, besides the SV40 intron, any intron supplying a fitting splice donor and splice acceptor site (minimal consensus sequences GT/AG) can serve as replacement intron. Respective introns are known in the art.

Preferably, the host cells used in the methods and uses of the present invention further have a transfer vector containing one or more GOIs flanked by AAV ITRs stably integrated into the host cell genome. Preferably, said host cells do not express VA RNA during the course of the methods and uses of the present invention, or express only low levels of VA RNA, as defined above.

Specifically, in preferred embodiments, the methods and uses of the present invention encompass the production of AAV particles as described above, culturing the host cells either adherently, on roller-bottles, on cell-stacks, or in fixed-bed bioreactors, or culturing the host cells in suspension in stir-tank bioreactors, wave or bag culture systems utilizing serum containing or serum free media. The scale of the production can vary from small volumes in the milliliter range over mid-range volumes up-to large scale volumes with 2000 L or 20 000 L volume per production run. The process type can be a batch run meaning without adding additional feed substance, or a fed-batch run with bolus or continues feed additions.

In order to achieve maximum titer process parameter like viable cell density, temperature, stirring speed, velocity, pH, and osmolarity can be changed during the production process. In addition, in order to avoid deprivation of certain amino acids, saccharides, organic acids, cofactors, vitamin, minerals, or other elements supplementation of the cell culture with the lacking components can be part of the production process.

Means for causing the expression of respective proteins in the host cells of the present invention are not particularly limited and are known in the art. They include for example the induction of inducible promoters by addition of suitable inducers.

All definitions and limitations defined for the first aspect of the present invention equally apply to the second and third aspect of the present invention.

As used herein, the term "comprising"/"comprises" expressly includes the terms "consisting essentially of"/"consists essentially of" and "consisting of"/"consists of", *i.e.,* all of said terms are interchangeable with each other.

In the present invention, the modification of the AAV capsid sequence enables stable producer cell lines with very low to no levels of VA RNA expression. Stable AAV producer cell lines have to express replicase, helper and capsid functions in a sufficient manner. Protein functions can be made inducible under the control of an inducible promoter.

In particular, the present invention advantageously identified a method to modify a 200 nucleotide region containing a PKR activating sequence in a way to maintain at the same time correct the expression pattern of VP proteins, e.g. by separating rep and cap and introducing a different intron. Therefore, it is possible to use the modified sequence in a stable cell line that expresses low levels of VA RNA to be enabled to produce high titer rAAV.

To circumvent the necessity of high levels of VA RNA, the endogenous p40 promoter and parts of the intron containing the sequence activating the protein kinase R (PKR) is replaced by other introns, such as e.g. SV40, beta-globin, chimeric introns in a manner that maintains the right ratio of VP proteins and at the same time does not activate the PKR, so less to no VA RNA is necessary for production of high levels of AAV vector.

This advantageously affords reproducible and consistent product quality, excellent scalability, cost-effective production, no necessity of helper virus, and the production of larger batches.

The figures show:
Figure 1: Schematic overview of capsid locus showing p40 promoter region with the p40 intron (SD: splice donor site, SA1: splice acceptor site 1, SA2: splice acceptor site 2). The upper drawing shows the wildtype configuration. Below the modified version indicating the replaced sequence. SD and SA1 are provided by the SV40 intron in this case; promoter can be a constitutive promoter as CMV or inducible as TRE3G.

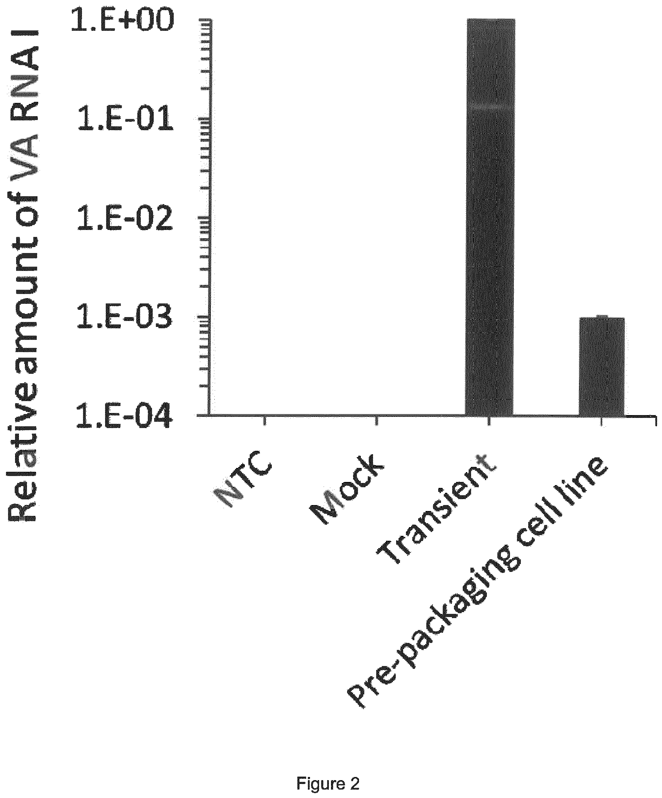

Figure 2: VA RNA levels in stable CAP pre-packaging cell line Z3081 harboring a stably integrated nucleic acid comprising VA RNA compared to a transient approach in which VA RNA was introduced via transient transfection encoded on a plasmid. Cells with stably integrated VA RNA display an up to 3 log reduced amount of VA RNA when compared to the transient approach.

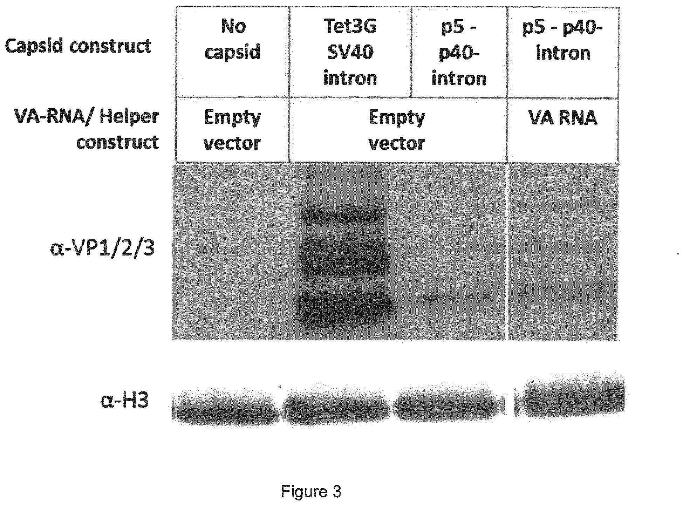

Figure 3: Expression of AAV8 capsid proteins in stable CAP pre-packaging SCC (single cell clone) upon transfection with capsid construct with p40 promoter or modified promoter with SV40 intron (TRE3G / SV40 intron) in combination with empty vector or additional VA RNA. 5h post-transfection cells were treated with 1mg/L doxycycline in order to induce the expression of rep, E2A, and E4orf6 protein. Protein levels were detected in cell lysates of transiently transfected pre-packaging cells 3 d post transfection using anti-VP1/VP2NP3 antibody (Progen).

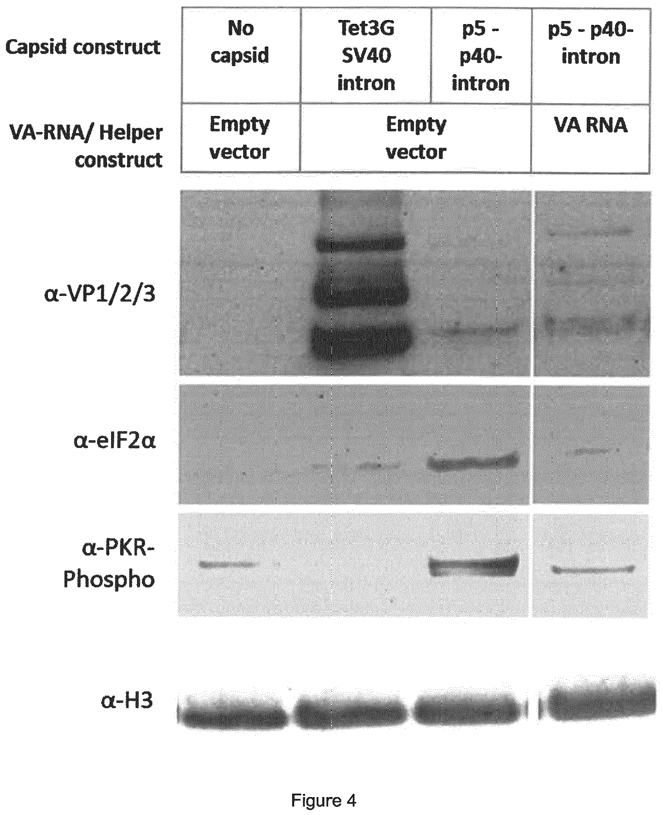

Figure 4: Activation of PKR and phosphorylation of eIF2α in stable CAP pre-packaging SCCs upon transfection with AAV8 capsid construct with p40 promoter or modified TRE3G promoter with SV40 intron in combination with empty vector (eV), or additional VA RNA. 5 h post transfection cells were treated with 1 mg/L doxycycline in order to induce the expression of the stably integrated replicase, E2A, and E4ORF6 protein. Protein levels were detected in cell lysates of transiently transfected CAP pre-packaging cells 3 d post transfection using anti-VP1/VP2/VP3 antibody (Progen), anti-Phospho-PKR antibody (Abcam), anti-eIF2α (Cell Signaling) and as loading control anti-Histone H3 antibody (Abeam). As control, cell lysate of empty vector-transfected cells was included.

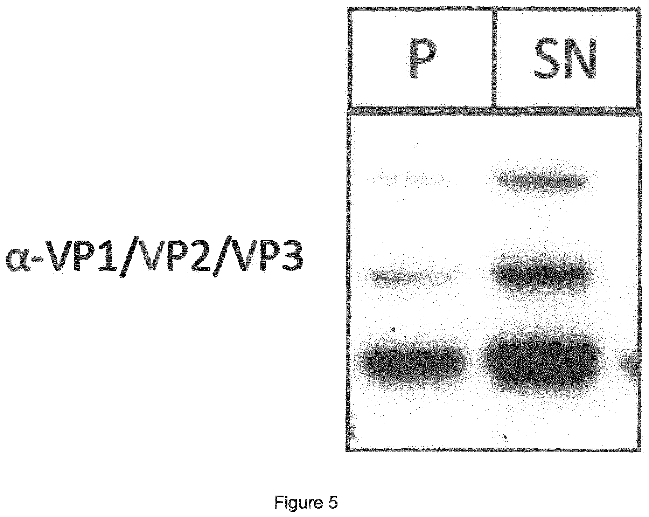

Figure 5: Expression of AAV8 capsid proteins in stable CAP pre-packaging SCC using the modified CMV / SV40 intron construct. Pre-packaging SCC#1 was transfected with CMV/SV40 intron modified capsid construct in combination with a GFP as Gol containing transfer construct and 5 h post-transfection induced with doxycycline. Protein levels were detected in cell lysates (P = pellet) and supernatant (SN) of transiently transfected CAP pre-packaging cells 4 d post transfection using anti-VP1/VP2/VP3 antibody (Progen).

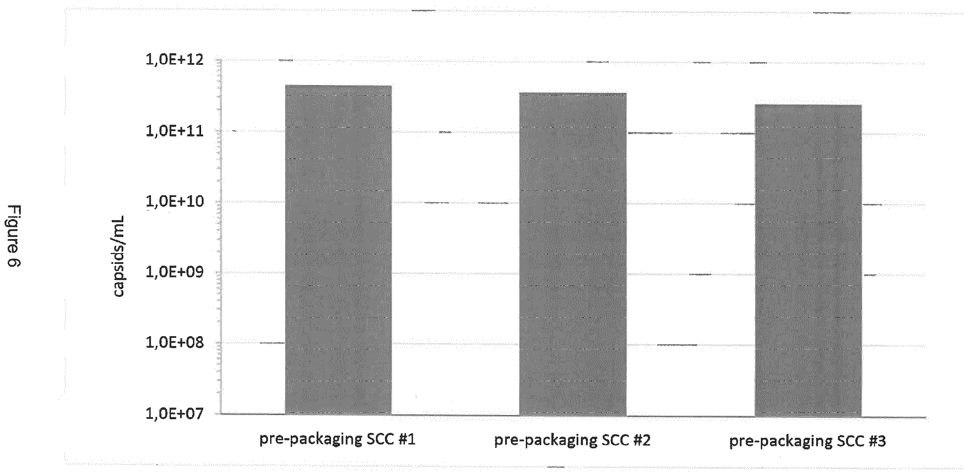

Figure 6: ELISA for assembled capsids (AAV8). Three different pre-packaging CAP single cell clones (SCC#1, SCC#2, SCC#3) were transfected with modified AAV8 capsid construct (CMV / SV40 intron) and AAV GOI construct (GFP) flanked by the ITRs. 5 h post transfection cells were induced with 1 mg/L doxycycline. 3 d post transfections, cell suspension samples were taken and analyzed for capsid expression using the AAV8 Progen capsid ELISA according to the manufacturer's instructions. This ELISA only detected fully assembled viral particle. High levels of assembled capsids above 10¹¹ particle/mL were detected.

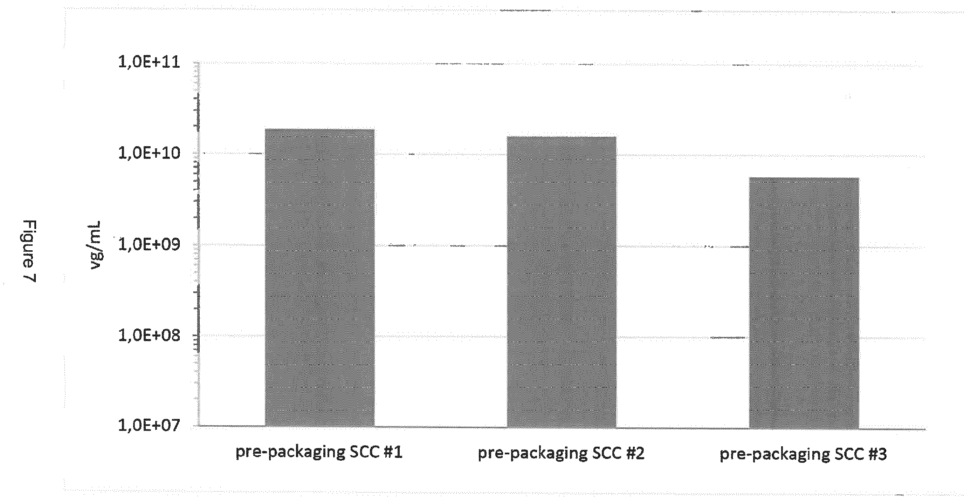

Figure 7: qPCR analysis of rAAV8-GFP production in pre-packaging SCCs (SCC#1, SCC#2, SCC#3) using the modified CMV / SV40 intron capsid construct. Three different pre-packaging SCCs were transfected with the modified capsid construct (CMV / SV40 intron) and GOI construct (GFP) flanked by the AAV2 ITRs. 5 h post transfection expression of the stably integrated replicase, and helper functions were induced by 1 mg/L doxycycline. 3 d post transfections, cell suspension samples were taken and analyzed for packaged viral genomes by qPCR using SV40 PolyA directed primer/probe.

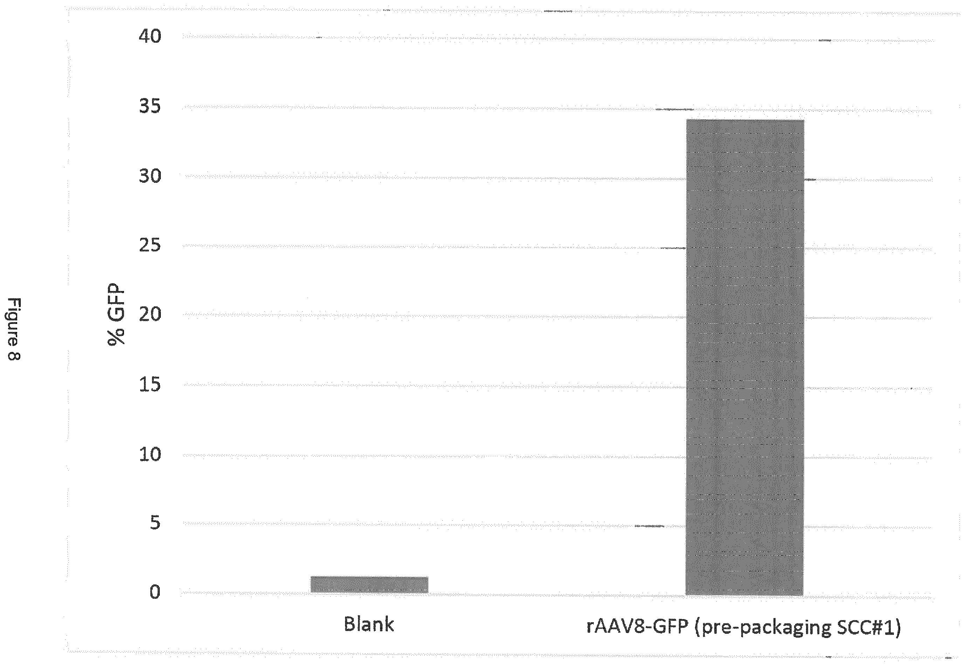

Figure 8: Transduction assay using material derived from pre-packaging SCC #1. Briefly, cell suspension from transfected pre-packaging SCC#1 was lysed by freeze-thaw cycles and used for transduction of HEK293T cells. Transduction was monitored by measurement of GFP positive HEK293T cells 48 h post transduction.

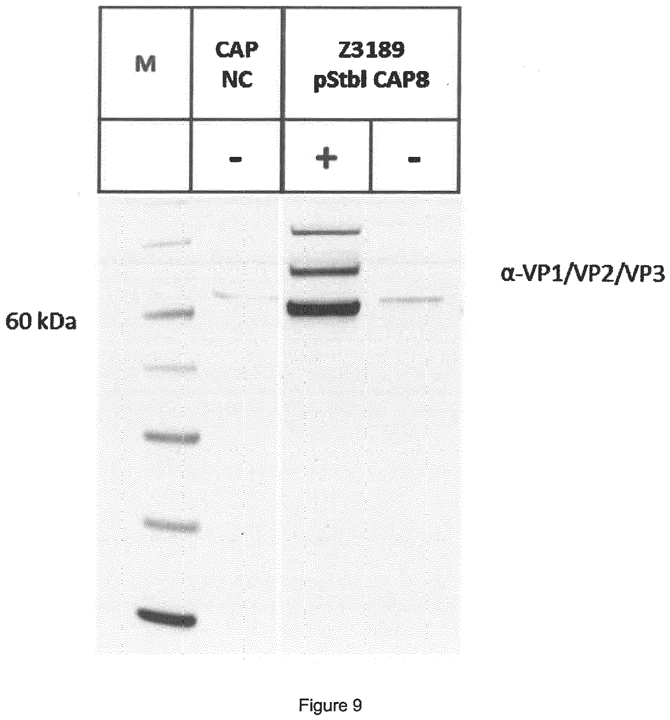

Figure 9: Western blot showing capsid expression (AAV8) in the correct stoichiometry in the cell lysate from the stable packaging pool Z3189 compromising stably integrated inducible replicase, helper functions and the capsid construct containing a modified inducible promoter as well as the SV40 intron upon doxycycline induction. Despite very low levels of VA RNA in the packaging pool cells capsid protein in the correct stoichiometry of approximate 1:1:10 for VP1:VP2: VP3 is produced.

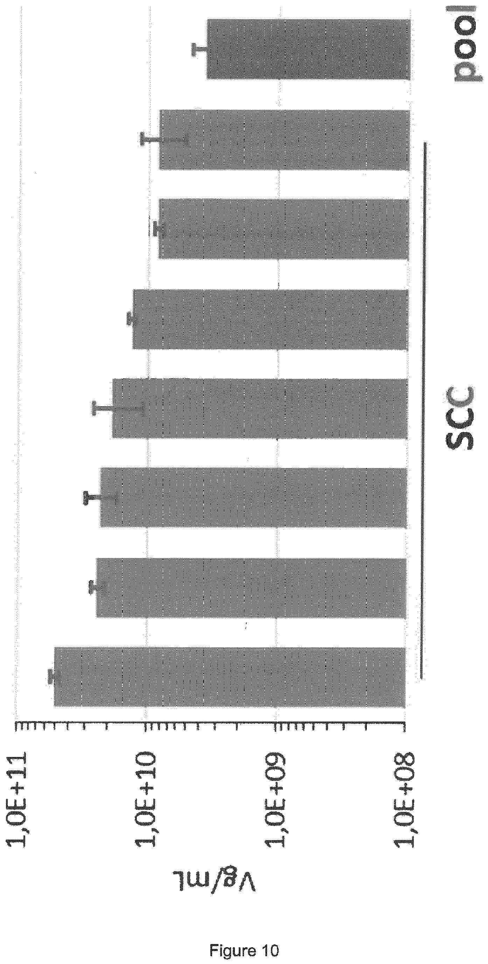

Figure 10: Viral genome production of selected CAP rAAV8-GFP producer single cell clones. AAV production in the CAP rAAV8 GFP producer SCC compromising stably integrated replicase, E2A, E4ORF6, VA RNA, the modified capsid construct (inducible promoter / SV40 intron), and the GOI flanked by the ITRs was induced by the addition of doxycycline. 7 d post induction cell suspension samples were taken and analyzed for packaged viral genomes by qPCR using SV40 PolyA directed primer/probe.

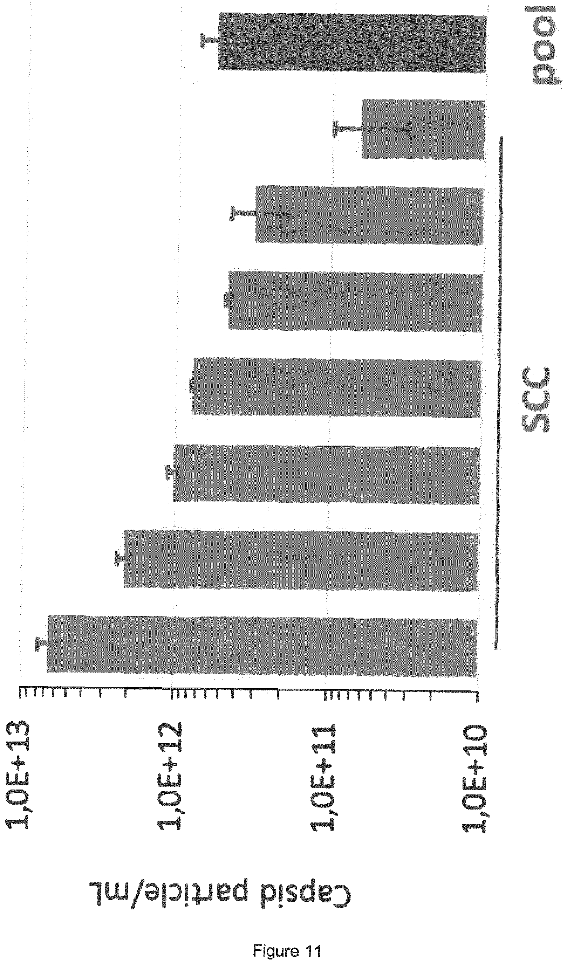

Figure 11: AAV8-ELISA analysis of selected CAP rAAV8-GFP producer single cell clones. AAV production in the CAP rAAV8 GFP producer SCC compromising stably integrated replicase, E2A, E4ORF6, VA RNA, the modified capsid construct, and the GOI flanked by the ITRs was induced by the addition of doxycycline. 7 d post induction, cell suspension samples were taken and analyzed for assembled capsids by using AAV8-ELISA (Progen) according to the manufacturer's instructions.

The present invention relates to the following amino acid and nucleotide sequences:
SEQ ID NO: 1
   SV40 PolyA primer forward
   AGCAATAGCATCACAAATTTCACAA
SEQ ID NO: 2
   SV40 PolyA primer reverse
   CCAGACATGATAAGATACATTGATGAGTT
SEQ ID NO: 3
   SV40 PolyA probe
   AGCATTTTTTTCACTGCATTCTAGTTGTGGTTTGTC

SEQ ID NO: 4
TRE3G-SV40intron-ATG of capsid
GAGTTTACTCCCTATCAGTGATAGAGAACGTATGAAGAGTTTACTCCCTATCAGTGATA
GAGAACGTATGCAGACTTTACTCCCTATCAGTGATAGAGAACGTATAAGGAGTTTACTC
CCTATCAGTGATAGAGAACGTATGACCAGTTTACTCCCTATCAGTGATAGAGAACGTAT
CTACAGTTTACTCCCTATCAGTGATAGAGAACGTATATCCAGTTTACTCCCTATCAGTG
ATAGAGAACGTATAAGCTTTAGGCGTGTACGGTGGGCGCCTATAAAAGCAGAGCTCGTT
TAGTGAACCGTCAGATCGCCTGGAGCAATTCCACAACACTTTTGTCTTATACCAACTTT
CCGTACCACTTCCTACCCTCGTAAAGACTCTAGAGGATCCGGTACTCGAGGAACTGAAA
AACCAGAAAGTTAACTGGTAAGTTTAGTCTTTTTGTCTTTTATTTCAGGTCCCGGATCC
GGTGGTGGTGCAAATCAAAGAACTGCTCCTCAGTGGATGTTGCCTTTACTTCTAGGCCT
GTACGGAAGTGTTACTTATG

### Promoter

### Intron

### Start codon

SEQ ID NO: 5
CMV-SV40intron-ATG of capsid

AATTCGAGCTTGCATGCCTGCAGGTCGTTACATAACTTACGGTAAATGGCCCGCCTGGC
TGACCGCCCAACGACCCCCGCCCATTGACGTCAATAATGACGTATGTTCCCATAGTAAC
GCCAATAGGGACTTTCCATTGACGTCAATGGGTGGAGTATTTACGGTAAACTGCCCACT
TGGCAGTACATCAAGTGTATCATATGCCAAGTACGCCCCCTATTGACGTCAATGACGGT
AAATGGCCCGCCTGGCATTATGCCCAGTACATGACCTTATGGGACTTTCCTACTTGGCA
GTACATCTACGTATTAGTCATCGCTATTACCATGGTGATGCGGTTTTGGCAGTACATCA
ATGGGCGTGGATAGCGGTTTGACTCACGGGGATTTCCAAGTCTCCACCCCATTGACGTC
AATGGGAGTTTGTTTTGGCACCAAAATCAACGGGACTTTCCAAAATGTCGTAACAACTC
CGCCCCATTGACGCAAATGGGCGGTAGGCGTGTACGGTGGGAGGTCTATATAAGCAGAG
CTCGTTTAGTGAACCGTCAGATCGCCTGGAGACGCCATCCACGCTGTTTTGACCTCCAT
AGAAGACACCGGGACCGATCCAGCCTCCGGACTCTAGAGGATCCGGTACTCGAGGAACT
GAAAAACCAGAAAGTTAACTGGTAAGTTTAGTCTTTTTGTCTTTTATTTCAGGTCCCGG
ATCCGGTGGTGGTGCAAATCAAAGAACTGCTCCTCAGTGGATGTTGCCTTTACTTCTAG
GCCTGTACGGAAGTGTTACTTCTGCTCTAAAAGCTGCGGAATTGTACCCGCGGCCGCTT
AATTAAATCGATACCGGTTTCGAAACGCGTGATAAATGCCACCATG

### Promoter

### Intron

### Start codon

The present invention will be further illustrated by the following examples without being limited thereto.

### Examples

### Experimental procedures

### Cell culture:

CAP cells and derived pre-packaging, packaging or producer cell lines were routinely cultivated in chemically defined, animal component-free PEM medium (Thermo Fisher Scientific) supplemented with 4 mM GlutaMAX (Gibco) in shake flasks (125 mL; Corning) on a shaking incubator at 120 - 185 rpm (5 cm orbit), 5 % CO₂ and 37 °C.

During routine cultivation, cells were diluted with fresh medium to a viable cell density of 0.5 - 1 × 10⁶ cells/ml every 72 - 96 h. Viable cell density and viability were determined by trypan blue exclusion using a CEDEX XS cell counter (Innovatis, Roche Applied Science) or ViCell Blu (Beckman Coulter).

### Nucleofection and generation of stable pools:

Stable pools were generated using Lonza's Nucleofector according to the manufacturer's instructions.

For each nucleofection reaction 1 × 10⁷ cells were harvested by centrifugation (500 × g, 5 min). The cells were resuspended in 100 µL complete nucleofector solution V (Lonza) and mixed with 5 µg of the linearized expression vector. The DNA/cell suspension was transferred into a cuvette and the nucleofection was performed using the X001 program. The transfected cells were transferred into 12.5 mL growth medium and cultured as described before at 37 °C, 5 % CO₂ at 185 rpm.

For generation of stable pools, cells were pelleted by centrifugation and resuspended in selection medium 72 - 96 h post-transfection following cultivation in a shaking incubator as described before.

**Table 1: Used cell lines**

| **Cell line** | **Components** |
|---|---|
| Pre-packaging cell line | Tet3G, inducible Rep, inducible Helper |
| Packaging cell line | Tet3G, inducible Rep, inducible Helper, inducible Capsid (incl. promoter / SV40 intron) |
| Producer cell line | Tet3G, inducible Rep, inducible Helper, inducible Capsid (incl. promoter / SV40 intron), GOI GFP |

### Transient transfection and western blot to test for capsid protein expression and PKR and EIF2a phosphorylation:

### Transient transfection:

Transient transfection was performed using PEImax (PolySciences) in FreeStyle 293 medium (Thermo Fisher Scientific). 5 h post transfection, cells were fed with complete PEM medium (Thermo Fisher Scientific) and expression of stably integrated replicase and helper functions were induced by addition of 1 µg/mL doxycycline. If not indicated otherwise, analysis of protein expression was performed 72 h post transfection.

### Western blot:

Western Blot analysis was performed with cell lysates from 1 × 10⁵ transfected cells, harvested 72 h post transfection and lysed using RIPA buffer supplemented with protease and phosphatase inhibitors. For western blot analysis the following antibodies were utilized: mouse-anti-VP1/NP2/NP3 antibody (Progen, Germany), anti-Phospho-PKR antibody (Abcam), anti-Phospho-eIF2α antibody (Cell Signaling) and horseradish peroxidase labeled anti-mouse and anti-rabbit antibody (Cell Signaling). Proteins were detected using the Pierce ECL WB Substrate Kit via chemiluminescence detector (INTAS).

### Viral production from pre-packaging, packaging, or producer cells with modified capsid construct:

To test for viral production using the modified capsid construct in presence of only the limited amount of VA RNA provided by the stable integration of the helper functions, the missing components necessary for the generation of AAV was introduced to the cells via transient transfections (Table 2). AAV production was initiated by inducing expression of replicase and helper proteins via doxycycline.

3 d post transfection, cell suspension samples were harvested and lysed by addition of a final concentration of 0.5 % Triton-X.

**Table 2: AAV production in stable cell lines**

| **Cell line** | **Stable integrated components** | **Complementary components for AAV production provided in trans** |
|---|---|---|
| Pre-packaging cell line | Tet3G, inducible Rep, inducible Helper | AAV8 capsid (CMV / SV40 intron), GOI GFP |
| Packaging cell line | Tet3G, inducible Rep, inducible Helper, inducible Capsid (ind. promoter / SV40 intron) | GOI GFP |
| Producer cell line | Tet3G, inducible Rep, inducible Helper, inducible Capsid (ind. promoter / SV40 intron), GOI GFP | / |

### qPCR to determine viral titer:

The following primer/dual-labelled probe combination (MWG, Eurofins; Table 3) directed against the SV40 PolyA was used to measure the viral titer:

**Table 3: Primer/Probe combination used for measuring the viral titer**

| **Primer/Probe** | **Sequence** |
|---|---|
| SV40 PolyA Primer for | 5'- AGC AAT AGC ATC ACA AAT TTC ACA A -3' (SEQ ID NO: 1) |
| SV40 PolyA Primer rev | 5'- CCA GAC ATG ATA AGA TAC ATT GAT GAG TT -3' (SEQ ID NO: 2) |
| SV40 PolyA Probe | Fam-5'-AGC ATT TTT TTC ACT GCA TTC TAG TTG TGG TTT GTC -3'-BHQ1 (SEQ ID NO: 3) |

| | |
|---|---|
| Fam: 6-Carboxyfluorescein; BHQ1: Black Hole Quencher 1 | |

As standard, linearized transgene plasmid with a defined copy number was used. The qPCR reaction contained the following components: 2 × Brilliant Multiplex qPCR Master Mix (Agilent), nuclease-free H₂O (Thermo Fisher Scientific), primer/probe mix and sample/standard. qPCR was run on an Agilent Mx3005P according to the manufacturer's instructions.

### ELISA for assembled capsids:

ELISA specific for assembled capsids was performed with commercially available ELISA Kits (Progen) according to the manufacturer's instructions.

### Assay for VA RNA Expression:

To test for VA RNA expression, RNA was isolated using the NucleoSpin miRNA kit according to the manufacturer's instructions. Following reverse transcription, analysis for VA RNA expression was performed using primer/probe combination specific for VA RNA and run on an Agilent Mx3005P according to the manufacturer's instructions.

### Transduction assay:

Briefly, cell lysates were prepared by 4 cycles of freeze-thaw. Adherent HEK293T cells were seeded and AAV particles were added. 48 h post transduction, transduced cells were quantified by GFP measurement.

### Example 1

### Influence of VA RNA on capsid expression

### Introduction:

CAP cells are human amniocyte-derived suspension cells, which have been immortalized by stable transfection with a construct encoding E1A/E1B. Pre-packaging cells are based on parental CAP cells and stably express (i) tet activator, (ii) replicase (AAV2) and adenoviral helper functions (E2A, E4orf6) under the control of a tet-inducible promoter and (iii) the adenoviral VA RNA. However, the stable integration of the adenoviral VA RNA with its PolIII RNA promoter into the genome of the stable pre-packaging cell lines results in very low levels of VA RNA (Figure 2), with an about of 3 log reduced RNA levels when compared to the transient transfection approach in which VA RNA is introduced to the cells on the pHelper plasmid.

VA RNA is important for high-titer AAV production. The function of the VA RNA within AAV production is the suppression of the PKR phosphorylation/activation and thereby, elF2alpha phosphorylation, which leads to a reduction of the protein expression of the viral replicase and capsid proteins. It has been shown that the PKR is activated by the sequence in the RNA leader sequence of the capsid proteins surrounding the AUG of VP1.

In the here described example the AAV8 capsid constructs contained either a modified capsid construct, comprising Tet3G promoter with SV40 intron (Tet3G/intron) or the P40 promoter construct, comprising the AAV2 p5 mini promoter as well as the AAV2 capsid p40 promoter with its intron (p5/p40/intron).

These were co-transfected with either VA RNA (construct only expressing the VA RNA), or empty vector serving as a control (Table 4). After induction of the stably integrated rep and helper genes via addition of doxycycline, cell lysates were prepared and analyzed for capsid expression.

**Table 4: Transient transfection of pre-packaging SCC to analyze AAV8 capsid expression and phosphorylation of PKR and eIF2α.**

| **Transfected** | **Co-transfected** |
|---|---|
| Capsid with modified intron/promoter | empty vector |
| Capsid with p40 promoter | empty vector |
| Capsid with p40 promoter | VA RNA |

### Result:

Pre-packaging cell lines have up to 3 log reduced VA RNA levels compared to transient approaches (Fig. 2). Interestingly, the capsid construct with the AAV p40 promoter and its intron region showed no expression without additional VA RNA construct, but an increase upon co-transfection of additional VA RNA functions. AAV8 capsid expression using the modified promoter construct compromising the TRE3G promoter and a SV40 intron could be detected even in the absence of additional VA RNA in pre-packaging SCC. In addition, due to the SV40 intron construct splicing can take place and therefore the correct ratio between the three different capsid proteins VP1:VP2:VP3 can be achieved (∼1:1:10). The correct ratio between the different VP is important for transduction efficiency of AAV, for the potency of the generated viral vector and therefore for its potential use a therapeutic gene therapy product (Fig. 3).

### Example 2:

### Effects of modification of capsid on PKR activation and expression level

### Introduction:

In pre-packaging cells, the expression of capsid with the original p40 promoter leads to very low expression level compared to using a modified capsid construct with a TRE3G promoter and a SV40 intron. However, additional expression of VA RNA increases titer (cf. Example 1). In order to test the hypothesis that due to the replacement of the PKR activating sequence in the RNA leader sequence of the capsid protein PKR activation will be diminished the following experiment was performed.

AAV8 capsid constructs containing either a modified capsid construct, comprising a TRE3G promoter with SV40 intron or the AAV p40 promoter construct, comprising p5 mini promoter, p40 promoter and its intron were co-transfected into pre-packaging cells in the presence or absence of additional plasmids encoding VA RNA (Table 4). After induction of the stably integrated rep and helper genes, cell lysates were prepared and analyzed for capsid expression and PKR and eIF2α phosphorylation.

### Result:

As shown before, the here described AAV pre-packaging cell lines have very low VA RNA levels compared to transient approaches (Fig. 2), which results in a decreased capsid protein titer in the cells using the AAV p5/p40/intron construct containing the PKR activating sequence. However, using the TRE3G / SV40 intron construct, involving the replacement of the PKR activating sequence of the native AAV promoter with another promoter is resulting in higher titers. Phosphorylation levels of the PKR and of the downstream target eIF2α, which have been described to reduce capsid expression, were indeed increased upon transfection of the p40 promoter and its intron containing capsid construct, but not when using the modified construct (Fig. 4).

Thus, replacement of the p40 promoter in combination with its intron by a modified TRE3G promoter in combination with a SV40 intron results in not only the correct ratio of the capsid proteins VP1:VP2:VP3, but also in increased protein expression due to reduced PKR activation and subsequent eIF2α phosphorylation.

### Example 3:

### AAV production using modified capsid construct

### Introduction:

Production of rAAV8-GFP was evaluated in three distinct pre-packaging SCCs by transfecting them with the modified capsid construct (CMV-promoter with SV40 intron) and construct with a GFP as GOI flanked by AAV2 ITRs. Transfection was done into the pre-packaging single cell clones already expressing inducible rep and helper functions, but as shown before extremely low levels of VA RNA. AAV production was initiated via doxycycline induction 5 h post-transfection.

Titers of assembled, intact AAV viral particles were analyzed via ELISA, vg/mL titer via qPCR utilizing SV40pA directed primer and probe. Infectivity was determined by transduction assay.

### Result:

The ELISA utilized in these studies recognizes a conformational epitope of assembled capsids. Therefore, the detection of above 1E11 particle/ml using the modified CMV / SV40 intron capsid construct in combination with the pre-packaging SCCs, reveals that fully assembled functional AAV particle with capsid proteins in the right stoichiometry are produced from these. These AAV particles are efficiently packaged with viral genomes as shown by qPCR. For the three different evaluated pre-packaging cells viral genome titer around 1E10/mL could be detected.

As VP1 is crucial for the infectivity of the AAV particle as the phospholipase A2 (PLA2) activity is required for the releasing of AAV particles from late endosomes, while VP2 and VP3 are crucial for correct virion assembly.

Analysis of the produced AAV particle in a transduction assay results in -34 % AAV transduced reporter cells and proves therefore the infectivity of the AAV particle generated by using the modified capsid construct. Again illustrating the production of capsid particle in which the right stoichiometry of VP1:VP2:VP3 could be achieved.

In conclusion, despite the extremely low levels of VA-RNA in the different pre-packaging SCCs fully functional AAV are generated by the here described approach (Figs. 5 to 8).

### Example 4:

### Generation of stable packaging cell line using a modified capsid promoter region

### Introduction:

Modified capsid construct under the control of an inducible promoter in combination with SV40 intron was introduced into pre-packaging SCC. Stable pools were generated by selection. After pool generation, cells were seeded with a density of 1 x 10⁶ cells/mL and capsid production was induced by addition of 1 µg/mL doxycycline. In addition, a non-induced control was included. 3 d post induction, cell lysates were prepared and capsid production in correct stoichiometry was analyzed by western blot.

### Result:

A stable packaging pool was generated using the modified capsid construct under the control of an inducible promoter based on a pre-packaging cell line. Upon doxycycline induction, the stable cell line expressed the capsid proteins despite low levels of VA RNA with high efficiency and in the right ratio as shown by western blot (Fig. 7).

### Example 5:

### Producer cell line

### Introduction:

A fully stable producer cell line was generated by introduction of the modified capsid construct under the control of an inducible promoter in combination with the SV40 intron and the ITR-flanked GOI GFP into pre-packaging SCC by lentiviral transduction. This polyclonal pool served as starting point for single cell cloning in order to obtain high producer cells. After identifying the most promising SCCs in small-scale 24 deep well plates experiments, the chosen SCCs were analyzed in an ambr run for rAAV8-GFP production upon induction. Shortly, cells were seeded with 1 x 10⁶ cells/mL and induced by addition of doxycycline 3 d post-seeding. Samples were taken at different time points and AAV production was measured by ELISA and qPCR.

### Result:

Using the modified capsid construct, stable producer cell lines were successfully generated. The best producer SCCs were analyzed for productivity in an ambr15 run. Upon induction, the fully stable producer cell lines showed high titer rAAV8-GFP production as detected by capsid ELISA and qPCR despite being based on pre-packaging clones having only low levels of VA RNA (Figs. 8 and 9). Capsid levels using the modified constructs were in the range of 10¹⁰ to 10¹³ capsids/mL depending on the SCC and viral genome production between mid 10⁹ range up to mid 10¹⁰.

## Claims

1. A host cell comprising
a nucleic acid encoding Adeno-associated virus (AAV) capsid protein, wherein the protein kinase R (PKR) activating sequence surrounding the start codon of AAV capsid protein VP1 is inactivated, while maintaining functional expression of the AAV capsid proteins VP1, VP2, and VP3.

2. The host cell according to claim 1, wherein said PKR-activating sequence is inactivated by replacing the splice donor and splice acceptor site 1 of the intron of the sequence encoding the capsid pre-mRNA.

3. The host cell according to claim 1 or claim 2, wherein said PKR-activating sequence is inactivated by replacing the native AAV capsid protein promoter p40 and the part of the p40 intron containing the splice donor and splice acceptor site 1 outside of the coding capsid sequence by an intron of different origin.

4. The host cell according to any one of claims 1 to 3, wherein the native AAV capsid protein promoter p40 is replaced by an inducible promoter, and/or the native AAV capsid protein promoter p40 intron is replaced outside of the capsid coding sequence by a replacement intron replacing the splice donor and splice acceptor site 1 and providing a fitting splice donor and splice acceptor site.

5. The host cell according to claim 4, wherein the replacement intron is selected from the group consisting of an SV40 intron, a synthetic CAG promoter intron, and a beta-globin intron.

6. The host cell according to any one of claims 1 to 5, further comprising
a nucleic acid encoding AAV replicase (Rep) proteins under the control of an inducible promoter, and/or
a nucleic acid encoding adenoviral E1A and E1B proteins, and/or
a nucleic acid encoding adenoviral E2A and E4orf6 proteins under the control of an inducible promoter.

7. The host cell according to any one of claims 1 to 6, further comprising a nucleic acid encoding a transfer vector containing one or more gene(s) of interest (GOI).

8. The host cell according to any one of claims 1 to 7, wherein the inducible promoter controlling expression of the AAV capsid protein and/or the inducible promoter controlling expression of AAV replicase proteins and/or the inducible promoter controlling expression of adenoviral E2A and E4orf6 proteins, is selected from the group consisting of a tet-inducible promoter, a cumate-inducible promoter, a tamoxifen-inducible promoter, a rapamycin-inducible promoter, an FKCsA-inducible promoter, an ABA-inducible promoter, riboswitch-controlled promoters, and heat shock promoter-driven, light-switchable systems.

9. The host cell according to any one of claims 1 to 8, wherein the PKR-activating sequence is inactivated by replacing the native AAV capsid protein promoter p40 and the part of the p40 intron containing the splice donor and splice acceptor site 1 outside of the coding capsid sequence by an intron of different origin,
wherein the native AAV capsid protein promoter p40 is replaced by a constitutive or inducible promoter, and the native AAV capsid protein promoter p40 intron is partially replaced, *i.e.,* is replaced outside of the capsid coding sequence, by an intron replacing the splice donor and splice acceptor site 1, and
wherein the respective nucleotide sequence from the inducible promoter to the start codon of the coding sequence of the AAV capsid protein has the structure
Promoter - N1 - Intron - N2 - ATG
wherein
Promoter is the constitutive or inducible promoter,
N1 is a sequence of 1 to 4000 nucleotides,
Intron is an intron, selected from the group consisting of introns supplying a fitting splice donor and splice acceptor site with the minimal consensus sequences GT/AG,
N2 is a sequence of 1 to 4000 nucleotides, and
ATG is the start codon of the AAV capsid protein coding sequence.

10. The host cell according to claim 9, wherein Promoter an inducible promoter, selected from the group consisting of a tet-inducible promoter such as the third generation TRE3G-promoter, a cumate-inducible promoter, a tamoxifen-inducible promoter, a rapamycin-inducible promoter, an FKCsA-inducible promoter, an ABA-inducible promoter, riboswitch-controlled promoters, and heat shock promoter-driven, light-switchable systems.

11. The host cell according to claim 9 or claim 10, wherein Intron is an intron, selected from the group consisting of a SV40 intron, a synthetic CAG promoter intron, and a beta-globin intron.

12. The host cell according to any one of claims 1 to 11, wherein the PKR-activating sequence is inactivated by replacing the native AAV capsid protein promoter p40 and the part of the p40 intron containing the splice donor and splice acceptor site 1 outside of the coding capsid sequence by an intron of different origin,
wherein the native AAV capsid protein promoter p40 is replaced by an inducible promoter, and the native AAV capsid protein promoter p40 intron is partially replaced, *i.e*., is replaced outside of the capsid coding sequence, by an SV40 intron, replacing the splice donor and splice acceptor site 1, and wherein the respective nucleotide sequence from the inducible promoter to the start codon of the coding sequence of the AAV capsid protein is
(i) the nucleotide sequence according to SEQ ID NO: 4 or SEQ ID NO: 5, or
(ii) a nucleotide sequence having at least 70% sequence identity to SEQ ID NO: 4 or SEQ ID NO: 5, provided the above prerequisites apply to said nucleotide sequence,
wherein the last three nucleotides ATG of SEQ ID NO: 4 or SEQ ID NO: 5 are the start codon of the AAV capsid protein coding sequence.

13. The host cell according to any one of claims 1 to 12, which is derived from a cell, selected from the group consisting of CAP cells, HEK293 cells, and Per.C6 cells.

14. A method for the production of Adeno-associated virus (AAV), comprising the step of expressing said AAV in a host cell according to any one of claims 1 to 13.

15. The use of a host cell according to any one of claims 1 to 13 in the production of Adeno-associated virus (AAV).


SEQUENCE LISTING

<110>  CEVEC Pharmaceuticals GmbH

<120>  Produced Cell Line Having Low Levels of VA RNA

<130>  C 4939EU - kl

<160>  5

<170>  PatentIn version 3.5

<210>  1
<211>  25
<212>  DNA
<213>  Artificial sequence

<220>
<223>  SV40 PolyA primer forward

<400>  1
agcaatagca tcacaaattt cacaa                                          25

<210>  2
<211>  29
<212>  DNA
<213>  Artificial sequence

<220>
<223>  SV40 PolyA primer reverse

<400>  2
ccagacatga taagatacat tgatgagtt                                      29

<210>  3
<211>  36
<212>  DNA
<213>  Artificial sequence

<220>
<223>  SV40 PolyA probe

<400>  3
agcatttttt tcactgcatt ctagttgtgg tttgtc                              36

<210>  4
<211>  551
<212>  DNA
<213>  Artificial sequence

<220>
<223>  TRE3G-SV40intron-ATG of capsid

<400>  4
gagtttactc cctatcagtg atagagaacg tatgaagagt ttactcccta tcagtgatag      60

agaacgtatg cagactttac tccctatcag tgatagagaa cgtataagga gtttactccc     120

tatcagtgat agagaacgta tgaccagttt actccctatc agtgatagag aacgtatcta     180

cagtttactc cctatcagtg atagagaacg tatatccagt ttactcccta tcagtgatag 240

agaacgtata agctttaggc gtgtacggtg ggcgcctata aaagcagagc tcgtttagtg 300

aaccgtcaga tcgcctggag caattccaca acacttttgt cttataccaa ctttccgtac 360

cacttcctac cctcgtaaag actctagagg atccggtact cgaggaactg aaaaaccaga 420

aagttaactg gtaagtttag tctttttgtc ttttatttca ggtcccggat ccggtggtgg 480

tgcaaatcaa agaactgctc ctcagtggat gttgccttta cttctaggcc tgtacggaag 540

tgttacttat g 551

<210> 5
<211> 872
<212> DNA
<213> Artificial sequence

<220>
<223> CMV-SV40intron-ATG of capsid

<400> 5
aattcgagct tgcatgcctg caggtcgtta cataacttac ggtaaatggc ccgcctggct 60

gaccgcccaa cgacccccgc ccattgacgt caataatgac gtatgttccc atagtaacgc 120

caatagggac tttccattga cgtcaatggg tggagtattt acggtaaact gcccacttgg 180

cagtacatca agtgtatcat atgccaagta cgccccctat tgacgtcaat gacggtaaat 240

ggcccgcctg gcattatgcc cagtacatga ccttatggga ctttcctact tggcagtaca 300

tctacgtatt agtcatcgct attaccatgg tgatgcggtt ttggcagtac atcaatgggc 360

gtggatagcg gtttgactca cggggatttc caagtctcca ccccattgac gtcaatggga 420

gtttgttttg gcaccaaaat caacgggact ttccaaaatg tcgtaacaac tccgccccat 480

tgacgcaaat gggcggtagg cgtgtacggt gggaggtcta tataagcaga gctcgtttag 540

tgaaccgtca gatcgcctgg agacgccatc cacgctgttt tgacctccat agaagacacc 600

gggaccgatc cagcctccgg actctagagg atccggtact cgaggaactg aaaaaccaga 660

aagttaactg gtaagtttag tctttttgtc ttttatttca ggtcccggat ccggtggtgg 720

tgcaaatcaa agaactgctc ctcagtggat gttgccttta cttctaggcc tgtacggaag 780

tgttacttct gctctaaaag ctgcggaatt gtacccgcgg ccgcttaatt aaatcgatac 840

cggtttcgaa acgcgtgata aatgccacca tg 872